(19) Europäisches Patentamt
European Patent Office
Office européen des brevets

(11) Veröffentlichungsnummer: **0 129 075**
**A2**

(12) # EUROPÄISCHE PATENTANMELDUNG

(21) Anmeldenummer: **84105676.5**

(22) Anmeldetag: **18.05.84**

(51) Int. Cl.³: **C 07 C 101/00**
C 07 C 99/00, C 07 C 125/065

(30) Priorität: **20.05.83 US 496775**

(43) Veröffentlichungstag der Anmeldung:
**27.12.84 Patentblatt 84/52**

(84) Benannte Vertragsstaaten:
**AT BE CH DE FR GB IT LI NL**

(71) Anmelder: **F. HOFFMANN-LA ROCHE & CO. Aktiengesellschaft**

**CH-4002 Basel(CH)**

(72) Erfinder: **Bolin, David Robert**
**111 Morris Avenue**
**Denville, N.J. 07834(US)**

(74) Vertreter: **Freiherr von Pechmann, Eckehart et al,**
**Patentanwälte Wuesthoff- v. Pechmann-Behrens-Goetz**
**Schweigerstrasse 2**
**D-8000 München 90(DE)**

(54) **Geschützte Aminosäurederivate und deren Herstellung.**

(57) Im wesentlichen 100%ig reine, am $N^{\alpha}$ durch einen Rest vom Urethan-Typus geschützte Aminosäuren und ein Verfahren für deren Herstellung werden beschrieben. Dieses Verfahren eliminiert die Bildung von Di und Tripeptid-Verunreinigungen; als Zwischenprodukte werden leicht hydrolysierbare Trimethylsilylester verwendet.

**EP 0 129 075 A2**

Croydon Printing Company Ltd.

PATENTANWÄLTE
WUESTHOFF - v. PECHMANN - BEHRENS - GOETZ
PROFESSIONAL REPRESENTATIVES BEFORE THE EUROPEAN PATENT OFFICE
MANDATAIRES AGRÉÉS PRÈS L'OFFICE EUROPÉEN DES BREVETS

D
SC
TE
TE
TE.

0129075



EP-58 285

RAN 4101/15

F.HOFFMANN-LA ROCHE & CO.

# Geschützte Aminosäurederivate und deren Herstellung

Die vorliegende Erfindung betrifft Aminosäurederivate und deren Herstellung. Präziser ausgedrückt betrifft die vorliegende Erfindung am $N^{\alpha}$ durch einen Rest vom Urethan-Typus geschützte Aminosäuren in im wesentlichen 100%ig reiner Form und ein Verfahren zu deren Herstellung.

Es hat sich gezeigt, dass manche am $N^{\alpha}$ durch einen Rest vom Urethan-Typus geschützte Aminosäuren, hergestellt gemäss der nachfolgenden Reaktion

$$R^2\text{-OCOCl} + H_2N\text{-}\overset{R^1}{\overset{|}{C}}H\text{-COOH} \longrightarrow R^2\text{-OCO-NH-}\overset{R^1}{\overset{|}{C}}H\text{-COOH} + HCl$$

worin $R^1$ die Seitenkette einer α-Aminosäure und
$R^2$ Alkyl oder Aralkyl bedeuten,

durch erhebliche Mengen (2-5% und mehr) der entsprechenden Di- und Tripeptide der Formel

$$H\text{-}(NH\text{-}\overset{R^1}{\overset{|}{C}}H\text{-CO})_x\text{-OH}$$

worin x 2 oder 3 bedeutet,

verunreinigt sind.

Bt/6.4.84

Im allgemeinen erfolgt die Reaktion in wässrig/organischen Lösungsmitteln in Gegenwart einer anorganischen Base ( wie Natriumcarbonat, Magnesiumoxid usw.) zwecks Neutralisation des gebildeten HCl (Schotten-Baumann). Während der Reaktion präsentiert sich die Carboxylgruppe als mit einem Metallkation koordiniertes Carboxylatanion, und die entsprechende Verbindung $R^2$-OCO-NH-CH($R^1$)-$COO^{\ominus}$ $M^{\oplus}$ kann mit einem zweiten Molekül des Reagens $R^2$-OCOCl reagieren, wobei ein reaktives gemischtes Anhydrid der allgemeinen Formel $R^2$-OCO-NH-CH($R^1$)-COOCOO$R^2$ gebildet wird.

Dieses Zwischenprodukt kann mit einem zweiten Aminosäuremolekül reagieren unter Bildung eines am $N^{\alpha}$ blockierten Dipeptids der Formel $R^2$-OCO-(NH-CH($R^1$)--$CO)_2$-$O^{\ominus}$ $M^{\oplus}$.

Bei Wiederholung der obigen Reaktionssequenz entstehen in abnehmenden Mengen höhere Homologe (Tripeptide, Tetrapeptide, usw.).

Die Mengen dieser als Verunreinigungen vorliegenden Peptide hängen von verschiedenen Faktoren ab, einschliesslich Typus des Acylierungsmittel, Base und Lösungsmittel sowie auch Temperatur und pH. Obschon Anstrengungen unternommen worden sind, die Verunreinigungen auf ein Minimum herabzusetzen, ist es bisher nicht möglich gewesen, diese zu eliminieren.

Um die unerwünschte Nebenreaktion zu verhindern, muss die Carboxylgruppe blockiert sein, vorzugsweise in Form eines labilen Esters. Es ist bekannt, dass Trimethylsilyl--("Tms")-Ester glatt gebildet und leicht wieder zu den Carbonsäuren hydrolysiert werden können. Barlos et al., J. Org. Chem. 47, 1324 (1982), benützten diese Tatsache in ihrer hohe Ausbeuten liefernden Synthese von $N^{\alpha}$-Tritylaminosäuren. Smithwick et al., J. Org. Chem. 39, 3441 (1974) machten von vorübergehendem Schutz durch Tms Gebrauch bei der Herstellung von $N^{\alpha}$,$N^{G,G}$-Tribenzyloxy-carbonyl-L-arginin, welches auf vorbekannten Wegen nur in geringen Ausbeuten erhalten werden konnte.

Das vorliegende Verfahren zur Herstellung von im wesentlichen 100%ig reinen, an der endständigen Aminogruppe geschützten Aminosäuren in hohen Ausbeuten umfasst

(a) Umsetzung einer Aminosäure mit Trimethylsilylchlorid in Gegenwart eines organischen Lösungsmittels und einer organischen Base;

(b) Umsetzung des erhaltenen Trimethylsilylesters mit einem die endständige Aminogruppe acylierenden Mittel unter Bildung eines Trimethylsilylesters einer am $N^{\alpha}$ durch einen Rest vom Urethan-Typus geschützten Aminosäure; und

(c) Hydrolyse des am $N^{\alpha}$ geschützten Aminosäuretrimethylsilylesters, wobei eine im wesentlichen 100%ig reine, an der endständigen Aminogruppe geschützte Aminosäure gebildet wird.

Die Methode ist sauber, schnell und auf verschiedene Schutzgruppen für die Seitenkette anwendbar. Die $N^{\alpha}$-(9-Fluorenylmethoxycarbonyl)-("Fmoc")-Derivate aller gängigen Aminosäuren sind nach dieser Methode hergestellt worden, und zwar in Ausbeuten von im allgemeinen über 95% und ohne nachweisbare Peptid-Verunreinigungen. Ausserdem ist diese Methode mit gleichermassen hervorragenden Ergebnissen mittels verschiedener anderer Schutzgruppen vom Urethan-Typus verwendet worden, wie tert.-Butoxycarbonyl ("Boc"), Carbobenzoxy ("Z") und 2,2,2-Trichloräthoxycarbonyl ("Troc").

Bei der Untersuchung verschiedener Fmoc-Aminosäuren (welche entweder im Handel erhältlich sind oder gemäss bekannten Methoden hergestellt wurden) ergaben sich erhebliche Verunreinigungen durch entsprechende Dipeptid-Derivate (siehe Tabelle I), wogegen gemäss dem vorliegenden Verfahren hergestellte Fmoc-Aminosäuren 100%ig rein waren (siehe Tabelle II).

Tabelle I

Peptid-Verunreinigungen in Fmoc-Aminosäuren

| | AA | AA-AA | AA-AA-AA |
|---|---|---|---|
| Fmoc-Gly-OH | 85,4 | 14,4 | 0,2 |
| | 97,7 | 2.3 | <0,18 |
| | 99,3 | 0.7 | <0.15 |
| Fmoc-Ala-OH | 95,8 | 4,2 | <0,11 |
| | 99,0 | 1,0 | <0,11 |
| Fmoc-Leu-OH | 98,01 | 1,99 | U.N.G. |
| Fmoc-Ile-OH | 100,0 | U.N.G. | U.N.G. |
| Fmoc-Glu(OtBu)-OH | 94,9 | 5,1 | N.B. |
| | 95,5 | 4.5 | N.B. |
| Fmoc-Lys(Boc)-OH | 98,4 | 1,6 | N.B. |
| Fmoc-Ser(tBu)-OH | 98,40 | 1,60 | U.N.G. |

AA = monomer
AA-AA = dimer
AA-AA-AA = trimer

U.N.G. = Unterhalb Nachweis-Grenze
N.B. = Nicht bestimmt

Tabelle ii

| | Ansatz | AA | | AA-AA | AA-AA-AA |
|---|---|---|---|---|---|
| | | Ausbeute | Reinheit | | |
| | [mMol] | [%] | [%] | [%] | [%] |
| Fmoc-Gly | 25 | 97,6 | 100 | 0 | 0 |
| Fmoc-Ala | 25 | 99,4 | 100 | 0 | 0 |
| Fmoc-Phe | 25 | 97 | 100 | 0 | 0 |
| Fmoc-Val | 25 | 94,1 | 100 | 0 | N.B. |
| Fmoc-Pro | 25 | 96 | 100 | 0 | N.B. |
| Fmoc-Gln | 25 | 100 | 100 | 0 | 0 |
| Fmoc-Met | 25 | 93 | 100 | 0 | N.B. |
| Fmoc-Trp | 25 | 98,5 | 100 | 0 | N.B. |
| Fmoc-Asp(OtBu) | 25 | 91,8 | 100 | 0 | N.B. |
| Fmoc-Leu | 25 | 92,3 | 100 | 0 | 0 |
| Fmoc-Ile | 25 | 94,4 | 100 | 0 | 0 |
| Fmoc-Glu(OtBu) | 25 | 94 | 100 | 0 | 0 |
| Fmoc-Lys(Boc) | 25 | 91 | 100 | 0 | N.B. |
| Fmoc-Ser(tBu) | 25 | 100 | 100 | 0 | 0 |
| Fmoc-Cys(tBu) | 25 | 97,3 | | | |
| Fmoc-Thr(tBu) | 25 | 100 | | | |

AA = monomer
AA-AA = dimer
AA-AA-AA = trimer
N.B. = Nicht bestimmt

Nach dem Verfahren gemäss vorliegender Erfindung wird die Aminosäure, welche vorzugsweise die L-Konfiguration hat, mit zwei Aequivalenten Tms-Cl in Methylenchlorid und in Gegenwart von Diisopropyläthylamin ("DIPEA") als Beispiel einer organischen Base umgesetzt. Wenn in der Seitenkette eine reaktive Funktion vorhanden ist, wie z.B. in Serin, werden drei Aequivalente verwendet.

$$H_2N\text{-}CH(R^1)\text{-}COOH + 2(H_3C)_3SiCl \xrightarrow[DIPEA]{CH_2Cl_2} (H_3C)_3Si\text{-}NH\text{-}CH(R^1)\text{-}COOSi(CH_3)_3$$

Das trimethylsilylierte Zwischenprodukt wird dann mit dem die endständige Aminogruppe acylierenden Agens umgesetzt, z.B. einem Derivat von Fmoc, Boc, Z oder Troc, vorzugsweise einem Chlorid, und dies ergibt den Trimethylsilylester der entsprechenden, am $N^\alpha$ durch einen Rest vom Urethan-Typus geschützten Aminosäure, z.B. den Fmoc--Aminosäure-Trimethylsilylester, welcher bei der Hydrolyse die am $N^\alpha$ durch einen Rest vom Urethan-Typus geschützte Aminosäure liefert, z.B. die Fmoc-Aminosäure, und zwar in hoher Ausbeute und frei von entsprechenden Di- und Tripeptidderivaten.

Die nachfolgende allgemeine Vorschrift kann zur Herstellung der im wesentlichen 100%ig reinen Fmoc-Aminosäuren verwendet werden, wogegen die nachfolgenden Beispiele das Verfahren gemäss vorliegender Erfindung weiter illustrieren. Die äusserst reinen Aminosäurederivate sind besonders geeignet als Reagenzien für automatisierte Peptid-Synthesen.

In einem 250-ml-Rundkolben werden 37,5 mMol fein gemahlene Aminosäure unter Stickstoff und unter heftigem Rühren in 87,5 ml destilliertem trockenem Methylenchlorid suspendiert. 9,52 ml (75 mMol) Tms-Cl werden in einer Portion zugegeben. Die Mischung wird während 1 Stunde am Rückfluss

gekocht und dann in einem Eisbad gekühlt. Nach sorgfältiger Zugabe von 11,3 ml (100 mMol) DIPEA wird in einer Portion Fmoc-Cl zugegeben. Die Lösung wird noch während 20 Minuten im Eisbad gerührt, worauf man sie sich auf Raumtemperatur erwärmen lässt. Nach 1-1,5 Stunden wird die Mischung konzentriert und dann zwischen 200 ml Diäthyläther und 250 ml 2,5%iger Natriumbicarbonatlösung verteilt. Die Phasen werden getrennt, und die wässrige Phase wird mit 2 x 50 ml Diäthyläther extrahiert. Die Aetherschichten werden mit 2 x 25 ml Wasser zurückgewaschen. Die vereinigten wässrigen Phasen werden mit 1,0N HCl bis zu einem pH von 2 angesäuert und mit 3 x 75 ml Essigsäureäthylester extrahiert. Die organische Phase wird über Natriumsulfat getrocknet, zu einem Oel eingeengt und durch azeotrope Destillation mit 2 x 30 ml Acetonitril getrocknet. Das Oel verfestigt sich im Hochvakuum, und geeignete Lösungsmittel können zur Umkristallisation des Produkts verwendet werden.

## Beispiel 1

### tert.-Butoxycarbonyl-L-alanin

2,67 g (30 mMol) L-Alanin werden in 87,5 ml Methylenchlorid suspendiert. Nach Zugabe von 10,78 ml (85 mMol) Tms-Cl wird die Mischung 1,5 Stunden am Rückfluss gekocht und dann auf 0°C abgekühlt. Dann werden 11,3 ml (100 mMol) DIPEA und anschliessend 5,46 g (25 mMol) Di-tert.-butyl--dicarbonat zugegeben. Man rührt die Mischung während 20 Minuten und lässt sie sich dann auf Raumtemperatur erwärmen. Nach 2 Stunden dampft man das Lösungsmittel ab und verteilt den Rückstand zwischen 50 ml 2,5%iger Natriumbicarbonatlösung und 50 ml Diäthyläther. Die wässrige Phase wird abgetrennt, mit 2 x 30 ml Aether gewaschen und mit Zitronensäurelösung bis zu einem pH von 2 angesäuert. Die erhaltene Mischung wird mit Natriumchlorid gesättigt und mit 3 x 40 ml Methylenchlorid extrahiert. Die vereinigten organischen Phasen werden vorsichtig mit 2 x 1 ml Wasser

gewaschen und getrocknet. Beim Eindampfen verbleiben 3,6 g (76%) eines weissen Festkörpers.

Aminosäureanalyse ergibt kein an der endständigen Aminogruppe geschütztes Ala-Ala oder Ala-Ala-Ala.

## Beispiel 2

N-Carbobenzoxy-L-thiazolidin-4-carbonsäure

3,33 g (25,0 mMol) L-Thiazolidin-4-carbonsäure werden in 75 ml trockenem Methylenchlorid suspendiert, worauf 6,35 ml (50,0 mMol) Tms-Cl zugegeben werden. Die Mischung wird während 1 Stunde unter heftigem Rühren am Rückfluss gekocht und dann in einem Eisbad auf 0°C abgekühlt, wonach langsam 11,0 ml (97,2 mMol) DIPEA zugegeben werden. Nach beendeter Zugabe werden 5,50 g (32,2 mMol) Carbobenzoxychlorid beigefügt. Die Mischung wird während 30 Minuten bei 0°C und dann während 1,5 Stunden bei Raumtemperatur gerührt. Dann dampft man das Lösungsmittel ab und verteilt den zurückbleibenden öligen Festkörper zwischen 100 ml 2,5%iger Natriumbicarbonatlösung und 50 ml Diäthyläther. Die wässrige Phase wird abgetrennt, mit 2 x 30 ml Aether extrahiert und mit 1,0N HCl bis zu einem pH von 2 angesäuert. Extraktion mit 3 x 50 ml Essigsäureäthylester und hierauf Trocknen und Eindampfen ergeben 6,0 g (90%) eines klaren Oels, welches gemäss Hochdruck-Flüssigkeitschromatographie homogen ist.

## Beispiel 3

2,2,2-Trichloräthoxycarbonyl-L-phenylalanin

4,96 g (30 mMol) L-Phenylalanin und 7,61 ml (60 mMol) Tms-Cl werden in 87,5 ml Methylenchlorid während 2 Stunden am Rückfluss gekocht. Die Mischung wird auf 0°C abgekühlt, worauf 5,30 g (25 mMol) Chlorameisensäure-trichloräthylester und 9,61 ml (85 mMol) DIPEA zugegeben werden. Man rührt während 20 Minuten bei 0°C und dann während 2 Stunden bei Raumtemperatur weiter. Dann dampft man das Lösungsmittel ab und löst den Rückstand in 2,5%iger Natriumbicarbonatlösung. Das wässrige Gemisch wird zweimal mit Diäthyläther extrahiert, mit 1N HCl bis zu einem pH von 4 angesäuert und dreimal mit Essigsäureäthylester extrahiert. Die vereinigten Essigsäureäthylester-Phasen werden getrocknet und eingedampft, und man erhält 8,1 g (95%) eines weissen Festkörpers. Umkristallisation aus Aether/Petroläther liefert kleine Nadeln vom Schmelzpunkt 129,5-131C.

Aminosäureanalyse ergibt kein an der endständigen Aminogruppe geschütztes Phe-Phe oder Phe-Phe-Phe.

# Patentansprüche

1. Im wesentlichen 100%ig reine $N^{\alpha}$-9-Fluorenylmethoxycarbonyl-Aminosäuren.

2. Trimethylsilylester von Aminosäuren mit einer $N^{\alpha}$-(9-Fluorenylmethoxycarbonyl)-Schutzgruppe.

3. Verbindungen nach einem der Ansprüche 1 und 2, worin die Aminosäure aus Glycin, Alanin, Phenylalanin, Valin, Prolin, Glutamin, Methionin, Tryptophan, Asparaginsäure, Leucin, Isoleucin, Glutaminsäure, Lysin, Serin, Asparagin, Cystein und Threonin ausgewählt ist.

4. Verbindungen nach einem der Ansprüche 1-3, worin die Aminosäure die L-Konfiguration aufweist.

5. Verfahren zur Herstellung von im wesentlichen 100%ig reinen, an der endständigen Aminogruppe geschützten Aminosäuren, dadurch gekennzeichnet, dass man

(a) eine Aminosäure in Gegenwart eines organischen Lösungsmittels und einer organischen Base mit Trimethylsilylchlorid umsetzt;

(b) den erhaltenen Trimethylsilylester durch Umsetzung mit einem die endständige Aminogruppe acylierenden Mittel in einen Trimethylsilylester einer am $N^{\alpha}$ durch einen Rest vom Urethan-Typus geschützten Aminosäure überführt; und

(c) den Trimethylsilylester der $N^{\alpha}$-geschützten Aminosäure unter Bildung einer im wesentlichen 100%ig reinen, an der endständigen Aminogruppe geschützten Aminosäure hydrolysiert.

6. Verfahren nach Anspruch 5, worin die Aminosäure aus Glycin, Alanin, Phenylalanin, Valin, Prolin, Glutamin, Methionin, Tryptophan, Asparaginsäure, Leucin, Isoleucin, Glutaminsäure, Lysin, Serin, Asparagin, Cystein und Threonin ausgewählt ist.

7. Verfahren nach Anspruch 5 oder 6, worin die Aminosäure die L-Konfiguration aufweist.

8. Verfahren nach einem der Ansprüche 5-7, worin das organische Lösungsmittel Methylenchlorid ist.

9. Verfahren nach einem der Ansprüche 5-8, worin die organische Base Diisopropyläthylamin ist.

10. Verfahren nach einem der Ansprüche 5-9, worin das Reagens zur Acylierung der endständigen Aminogruppe ein 9-Fluorenylmethoxycarbonyl-, tert.-Butoxycarbonyl-, Carbobenzoxy- oder 2,2,2-Trichloräthoxycarbonyl-Derivat ist.

11. Verfahren nach Anspruch 10, worin das 9-Fluorenylmethoxycarbonyl-Derivat 9-Fluorenylmethoxycarbonylchlorid ist.

12. Verfahren nach einem der Ansprüche 5-11, worin die Aminosäure mit mindestens zwei Moläquivalenten von Trimethylsilylchlorid umgesetzt wird.

***

Patentansprüche für Oesterreich

1. Verfahren zur Herstellung von im wesentlichen 100%ig reinen, an der endständigen Aminogruppe geschützten Aminosäuren, dadurch gekennzeichnet, dass man

(a) eine Aminosäure in Gegenwart eines organischen Lösungsmittels und einer organischen Base mit Trimethylsilylchlorid umsetzt;

(b) den erhaltenen Trimethylsilylester durch Umsetzung mit einem die endständige Aminogruppe acylierenden Mittel in einen Trimethylsilylester einer am $N^{\alpha}$ durch einen Rest vom Urethan-Typus geschützten Aminosäure überführt; und

(c) den Trimethylsilylester der $N^{\alpha}$-geschützten Aminosäure unter Bildung einer im wesentlichen 100%ig reinen, an der endständigen Aminogruppe geschützten Aminosäure hydrolysiert.

2. Verfahren nach Anspruch 1, worin die Aminosäure aus Glycin, Alanin, Phenylalanin, Valin, Prolin, Glutamin, Methionin, Tryptophan, Asparginsäure, Leucin, Isoleucin, Glutaminsäure, Lysin, Serin, Asparagin, Cystein und Threonin ausgewählt ist.

3. Verfahren nach Anspruch 1 oder 2, worin die Aminosäure die L-Konfiguration aufweist.

4. Verfahren nach einem der Ansprüche 1-3, worin das organische Lösungsmittel Methylenchlorid ist.

5. Verfahren nach einem der Ansprüche 1-4, worin die organische Base Diisopropyläthylamin ist.

6. Verfahren nach einem der Ansprüche 1-5, worin das Reagens zur Acylierung der endständigen Aminogruppe ein 9-Fluorenylmethoxycarbonyl-, tert.-Butoxycarbonyl-, Carbobenzoxy- oder 2,2,2-Trichloräthoxycarbonyl-Derivat ist.

7. Verfahren nach Anspruch 6, worin das 9-Fluorenylmethoxycarbonyl-Derivat 9-Fluorenylmethoxycarbonylchlorid ist.

8. Verfahren nach einem der Ansprüche 1-7, worin die Aminosäure mit mindestens zwei Moläquivalenten von Trimethylsilylchlorid umgesetzt wird.

***